(19) Europäisches Patentamt
European Patent Office
Office européen des brevets

(11) **EP 3 239 876 B1**

(12) **EUROPEAN PATENT SPECIFICATION**

(45) Date of publication and mention
of the grant of the patent:
**07.08.2024 Bulletin 2024/32**

(21) Application number: **16189424.1**

(22) Date of filing: **19.09.2016**

(51) International Patent Classification (IPC):
**G16B 30/00** *(2019.01)*    **G16B 40/30** *(2019.01)*

(52) Cooperative Patent Classification (CPC):
**G16B 30/00; G16B 40/30**

(54) **METHOD AND SYSTEM FOR REPRESENTING COMPOSITIONAL PROPERTIES OF A BIOLOGICAL SEQUENCE FRAGMENT AND APPLICATIONS THEREOF**

VERFAHREN UND SYSTEM ZUR DARSTELLUNG DER ZUSAMMENSETZUNGSEIGENSCHAFTEN EINES FRAGMENTS EINER BIOLOGISCHEN SEQUENZ UND ANWENDUNGEN DAVON

PROCÉDÉ ET SYSTÈME DE REPRÉSENTATION DE PROPRIÉTÉS DE COMPOSITION D'UN FRAGMENT DE SÉQUENCE BIOLOGIQUE ET LEURS APPLICATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2016 IN 201621014353**

(43) Date of publication of application:
**01.11.2017 Bulletin 2017/44**

(73) Proprietor: **Tata Consultancy Services Limited Maharashtra (IN)**

(72) Inventors:
- MANDE, Sharmila Shekhar
  411 013 Pune - Maharashtra (IN)
- HAQUE, Mohammed Monzoorul
  411 013 Pune - Maharashtra (IN)
- BOSE, Tungadri
  411 013 Pune - Maharashtra (IN)
- DUTTA, Anirban
  411 013 Pune - Maharashtra (IN)
- CHENNAREDDY, Venkata Sivakumar Reddy
  411 013 Pune - Maharashtra (IN)

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**EP-A2- 2 626 802**

- **MOHAMMED MONZOORUL HAQUE ET AL: "CS-SCORE: Rapid identification and removal of human genome contaminants from metagenomic datasets", GENOMICS, vol. 106, no. 2, 1 August 2015 (2015-08-01), US, pages 116 - 121, XP055405512, ISSN: 0888-7543, DOI: 10.1016/j.ygeno.2015.04.005**
- **ANA B. FERNANDEZ ET AL: "Comparison of prokaryotic community structure from Mediterranean and Atlantic saltern concentrator ponds by a metagenomic approach", FRONTIERS IN MICROBIOLOGY, vol. 5, 8 May 2014 (2014-05-08), XP055405930, DOI: 10.3389/fmicb.2014.00196**

**Description**

CROSS-REFERENCE TO RELATED APPLICATIONS AND PRIORITY

The present application claims priority from Indian non-provisional specification no. 201621014353 filed on 25th April, 2016.

TECHNICAL FIELD

The present application generally relates to computing a numerical score for any given biological sequence. Particularly, the application relates to representing compositional properties of biological sequences using computed numerical score. More particularly, the application provides a method and system for representing compositional properties of a biological sequence fragment using a unidimensional compositional metric, wherein the computed metric finds utility in various genomic and metagenomic applications which involve comparison, categorization and/ or annotation of multiple biological sequences.

BACKGROUND

Current generation of sequencing platforms can generate millions of biological sequences in a single overnight run. Consequently, categorization and/or biological annotation of these sequences requires comparison of the generated biological sequences either amongst themselves or with sequences listed in existing sequence databases.
A majority of existing biological sequence comparison solutions rely on employing sequence alignment or sequence composition-based procedures. However, the alignment-based comparison of multiple biological sequences represents a NP-hard problem. Some of the prior art literature also describe about sequence composition-based procedures for comparison of biological sequences based on one or more compositional properties, which is/are represented typically in form of multidimensional vectors. However, analyzing large volumes of biological sequences using either of these procedures is typically compute intensive making real-time data analysis a significant challenge.
It is expected that comparison between biological sequences represented using a compositional metric that has 'fewer' dimensions would be relatively less compute intensive as compared to using a compositional metric that has a 'higher' number of dimensions. Most of the existing dimensionality reduction techniques such as PCA, MDS perform dimensionality reduction by decomposing the original dimensions in a dataset and creating a smaller number of entirely new dimensions to describe the data. Therefore, while comparing multiple datasets by employing existing dimensional reduction techniques, it becomes necessary to merge all the compared datasets prior to proceeding with the 'dimensionality reduction' and subsequent analysis. This renders the overall comparison procedure even more compute intensive with increasing number of datasets.
Prior art literature have illustrated various methods and techniques for biological sequence comparison, however, designing a method and system for representing compositional properties of a biological sequence fragment using a compositional metric with minimum number of dimensions, such as one, i.e. unidimensional, to be used for various genomic and metagenomic applications involving comparison of multiple biological sequences, is a significant technical challenge.

SUMMARY

The present invention provides a computer implemented method as defined in claim 1.
The present invention also provides a system as defined in claim 10.
In another embodiment of the invention, a non-transitory computer-readable medium having embodied thereon a computer program as defined in claim 11 is provided.

BRIEF DESCRIPTION OF ACCOMPANYING DRAWINGS

The foregoing summary, as well as the following detailed description, are better understood when read in conjunction with the appended drawings. For the purpose of illustrating the invention, there is shown in the drawings constructions of the invention. In the drawings:

Figure 1: shows a flow chart illustrating a method for representing compositional properties of a biological sequence fragment;
Figure 2: shows a block diagram illustrating system architecture for representing compositional properties of a biological sequence fragment; and

Figure 3: shows a flow chart illustrating a method for representing compositional properties of a biological sequence fragment in an embodiment that exemplifies an application of the depicted method in the field of metagenomics.

The Figures depict various embodiments of the present invention for purposes of illustration only. One skilled in the art will readily recognize from the following discussion that alternative embodiments of the structures and methods illustrated herein may be employed without departing from the principles of the invention described herein.

DETAILED DESCRIPTION OF THE INVENTION

Some embodiments of this invention, illustrating all its features, will now be discussed in detail.

The words "comprising," "having," "containing," and "including," and other forms thereof, are intended to be equivalent in meaning and be open ended in that an item or items following any one of these words is not meant to be an exhaustive listing of such item or items, or meant to be limited to only the listed item or items.

It must also be noted that as used herein and in the appended claims, the singular forms "a," "an," and "the" include plural references unless the context clearly dictates otherwise. Although any systems and methods similar or equivalent to those described herein can be used in the practice or testing of embodiments of the present invention, the preferred, systems and methods are now described.

The elements illustrated in the Figures inter-operate as explained in more detail below. For example, although selected aspects, features, or components of the implementations are depicted as being stored in memories, all or part of the systems and methods consistent with the attrition warning system and method may be stored on, distributed across, or read from other machine-readable media.

The techniques described above may be implemented in one or more computer programs executing on (or executable by) a programmable computer including any appropriate combination of any appropriate number of the following: a processor, a storage medium readable and/or writable by the processor (including, for example, volatile and non-volatile memory and/or storage elements), plurality of input units, and plurality of output devices. Program code may be applied to input entered using any of the plurality of input units to perform the functions described and to generate an output displayed upon any of the plurality of output devices.

Each computer program within the scope of the claims below may be implemented in any programming language, such as assembly language, machine language, a high-level procedural programming language, or an object-oriented programming language. The programming language may, for example, be a compiled or interpreted programming language. Each such computer program may be implemented in a computer program product tangibly embodied in a machine-readable storage device for execution by a computer processor.

Method steps of the invention may be performed by one or more computer processors executing a program tangibly embodied on a computer-readable medium to perform functions of the invention by operating on input and generating output. Suitable processors include, by way of example, both general and special purpose microprocessors. Generally, the processor receives (reads) instructions and data from a memory (such as a read-only memory and/or a random access memory) and writes (stores) instructions and data to the memory. Storage devices suitable for tangibly embodying computer program instructions and data include, for example, all forms of non-volatile memory, such as semiconductor memory devices, including EPROM, EEPROM, and flash memory devices; magnetic disks such as internal hard disks and removable disks; magneto-optical disks; and CD-ROMs. Any of the foregoing may be supplemented by, or incorporated in, specially-designed ASICs (application-specific integrated circuits) or FPGAs (Field-Programmable Gate Arrays). A computer can generally also receive (read) programs and data from, and write (store) programs and data to, a non-transitory computer-readable storage medium such as an internal disk (not shown) or a removable disk.

Any data disclosed herein may be implemented, for example, in one or more data structures tangibly stored on a non-transitory computer-readable medium. Embodiments of the invention may store such data in such data structure(s) and read such data from such data structure(s).

The present application provides a computer implemented method and system for representing compositional properties of a biological sequence fragment using a unidimensional compositional metric.

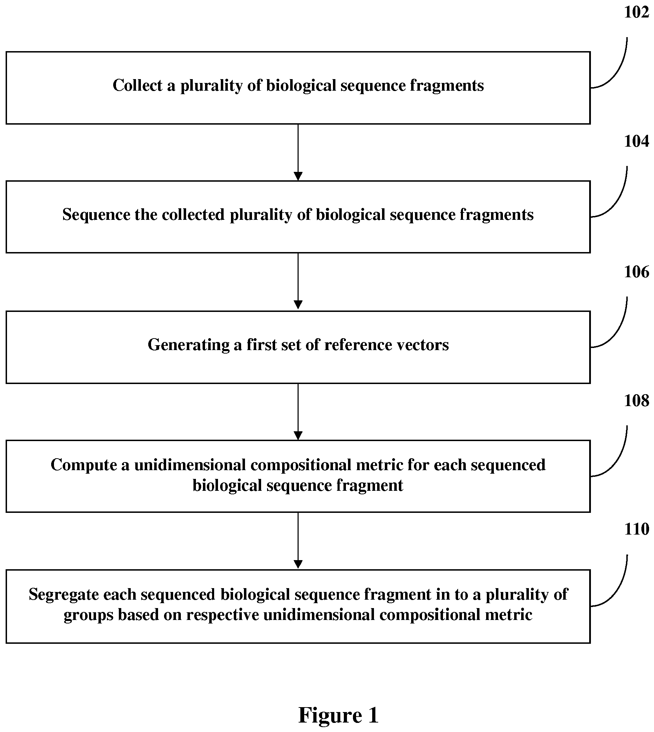

Referring to Figure 1 is a flow chart illustrating a method for representing compositional properties of a biological sequence fragment.

The process starts at step 102, a plurality of biological sequence fragments are collected. At the step 104, the collected plurality of biological sequence fragments are sequenced. At the step 106, a first set of reference vectors is generated, by generating a 256-dimensional tetra-nucleotide frequency vector (v) corresponding to the each sequenced biological sequence fragment out of the plurality of sequenced biological sequence fragments wherein the 256-dimensional tetra-nucleotide frequency vectors are subjected to Principal Component Analysis (PCA); selecting two vectors that lie at the extremes of the first principal component, i.e. the two selected vectors are maximally separated along PC1 (principal component 1); repeating selection of two discrete vectors each for PC2, PC3, ..., PCn so as to select two discrete vectors in each iteration, proceeding in the order of PC1, PC2, PC3 .... PCn, for generating a first set of reference vectors, wherein the first set of reference vectors comprises of the discrete vector pairs arranged in the order of their selection, i.e. in an order in which the reference vector pairs derived from the extremes of the most significant principal components precede reference vector pairs derived from the extremes of relatively less significant principal components.

At the step 108, a unidimensional compositional metric is computed for each sequenced biological sequence fragment out of the plurality of sequenced biological sequence fragments as a cumulative function of the distance of the tetra-nucleotide frequency vector (v) corresponding to an individual biological sequence fragment, from three or more reference vectors selected out of the generated first set of reference vectors. The process ends at the step 110, each sequenced biological sequence fragment out of the plurality of sequenced biological sequence fragments is segregated in to a plurality of groups based on respective unidimensional compositional metric.

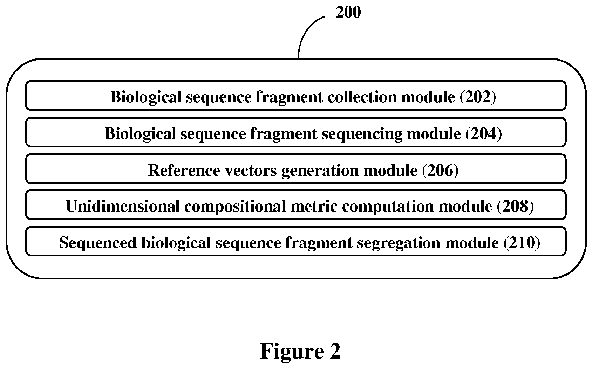

Referring to Figure 2 is a block diagram illustrating system architecture for representing compositional properties of a biological sequence fragment.

In an embodiment of the present invention, a system (200) is provided for representing compositional properties of a biological sequence fragment using a unidimensional compositional metric.

The system (200) for representing compositional properties of a biological sequence fragment using a unidimensional compositional metric comprising a processor; a data bus coupled to said processor; a computer-usable medium embodying computer code, said computer-usable medium being coupled to said data bus, said computer program code comprising instructions executable by said processor and configured for executing a biological sequence fragment collection module (202); a biological sequence fragment sequencing module (204); a reference vectors generation module (206); a unidimensional compositional metric computation module (208); and a sequenced biological sequence fragment segregation module (210)

In another embodiment of the present invention, the biological sequence fragment collection module (202) is adapted for collecting a plurality of biological sequence fragments. The plurality of biological sequence fragments are collected from a group comprising of genomic and/ or metagenomic and/ or environmental samples.

In another embodiment of the present invention, the biological sequence fragment sequencing module (204) is adapted for sequencing the collected plurality of biological sequence fragments.

In another embodiment of the present invention, the reference vectors generation module (206) is adapted for generating a 256-dimensional tetra-nucleotide frequency vector (v) corresponding to each sequenced biological sequence fragment out of the plurality of sequenced biological sequence fragments wherein the entire set of 256-dimensional tetra-nucleotide frequency vectors so generated are subjected to Principal Component Analysis (PCA). Further, two vectors that lie at the extremes of the first principal component i.e. maximally separated along PC1 (principal component 1) are first selected. Furthermore, selection of two vectors is repeated for PC2, PC3, ..., PCn such that two discrete vectors are selected in each iteration, proceeding in the order of PC1, PC2, PC3 .... PCn, for generating a first set of reference vectors, wherein the first set of reference vectors comprises of the discrete vector pairs arranged in the order of their selection, i.e. in an order in which the reference vector pairs derived from the extremes of the most significant principal components precede reference vector pairs derived from the extremes of relatively less significant principal components. Given that each of the principal components are orthogonal to each other, the first set of reference vectors (rv1, rv2, rv3, .., rvN) generated at the end of this step, are sufficiently separated from each other in the 256 dimensional space.

In an alternative embodiment of the present invention, the reference vectors generation module (206) is adapted for generating n-dimensional frequency vector for a plurality of k-mer frequencies wherein the plurality of k-mer frequencies are other than tetra-nucleotide frequency. The frequency vectors for other k-mer frequencies may also be generated, i.e. other than tetra nucleotide frequencies and therefore the dimensionality of the feature vector space may be other than 256 dimensions.

The distance between the 256-dimensional tetra-nucleotide frequency vector (v) corresponding to the each sequenced biological sequence fragment out of the plurality of sequenced biological sequence fragments is computed using a distance metric. The distance metric used to compute the distance between the 256-dimensional tetra-nucleotide frequency vector (v) corresponding to the each sequenced biological sequence fragment out of the plurality of sequenced biological sequence fragments is selected from a group comprising but not limited to Manhattan distance or Euclidean distance or an appropriate metric suitable for measuring distance in a multidimensional space.

In another embodiment of the present invention, the unidimensional compositional metric computation module (208) is adapted for computing a unidimensional compositional metric for each sequenced biological sequence fragment out of the plurality of sequenced biological sequence fragments as a cumulative function of the distance of the tetra-nucleotide frequency vector (v) corresponding to an individual biological sequence fragment, from the first three or more reference vectors (rv1, rv2, rv3, .., rvN) selected out of the generated first set of reference vectors. The unidimensional compositional metric is cmp-score, which is computed according to the following:

$$\text{cmp-score} = \text{dist}(v\text{-}rv1) + \text{dist}(v\text{-}rv2) + \text{dist}(v\text{-}rv3) + ... + \text{dist}(v\text{-}rvN)$$

In another embodiment of the present invention, the sequenced biological sequence fragment segregation module (210) is adapted for segregating each sequenced biological sequence fragment out of the plurality of sequenced biological sequence fragments into a plurality of groups based on respective computed unidimensional compositional metric.

The resulting groups, each comprising one or more sequenced biological sequence fragment(s) amongst the plurality of sequenced biological sequence fragments, formed on the basis of respective computed unidimensional compositional metric, are utilized in genomic and/ or metagenomic sequence analysis applications which involve/ require rapid ordering, comparison, categorization, and annotation of each sequenced biological sequence fragment out of the plurality of sequenced biological sequence fragments.

In an alternative embodiment of the present invention, the computing of the unidimensional compositional metric for each sequenced biological sequence fragment out of the plurality of sequenced biological sequence fragments as a cumulative function of the distance of the tetra-nucleotide frequency vector (v) from three or more reference vectors, wherein the three or more reference vectors are derived from a second set of reference vectors.

The derivation of the second set of reference vectors comprising steps of generating a 256-dimensional tetra-nucleotide frequency vector (v) corresponding to each of a plurality of randomly generated biological sequence fragments of a predetermined length. Wherein, the length of the plurality of randomly generated biological sequence fragments may be determined based on the average length of query sequence(s) for which cmp-score needs to be generated. The plurality of randomly generated biological sequence fragments are derived from completely sequenced genomes. For each of these sequence fragments, vectors representing the frequencies of all possible tetra-nucleotides (in that sequence) are computed. The entire set of 256-dimensional tetra-nucleotide frequency vectors are subjected to Principal Component Analysis (PCA). Further, two vectors that lie at the extremes of the first principal component i.e. maximally separated along PC1 (principal component 1) are first selected. Furthermore, selection of two vectors is repeated for PC2, PC3, ..., PCn, such that two discrete vectors are selected in each iteration, proceeding in the order of PC1, PC2, PC3 .... PCn, for generating a second set of reference vectors, wherein the second set of reference vectors comprises of the discrete vector pairs arranged in the order of their selection, i.e. in an order in which the reference vector pairs derived from the extremes of the most significant principal components precede reference vector pairs derived from the extremes of relatively less significant principal components. Given that each of the principal components are orthogonal to each other, the reference vectors comprising the second set of reference vectors are sufficiently separated from each other in the 256 dimensional space.

The 256-dimensional tetra-nucleotide frequency vector (v) corresponding to the each sequenced biological sequence fragment out of the plurality of sequenced biological sequence fragments generation is a one-time process and may not be repeated before proceeding to subsequent steps of the method and system for representing compositional properties of the biological sequence fragment using the unidimensional compositional metric. Further, the reference vector set generated from one set of biological sequences may be employed for generating cmp-scores for any biological sequence fragment either from the current study or experiment as well as from any other study or experiment.

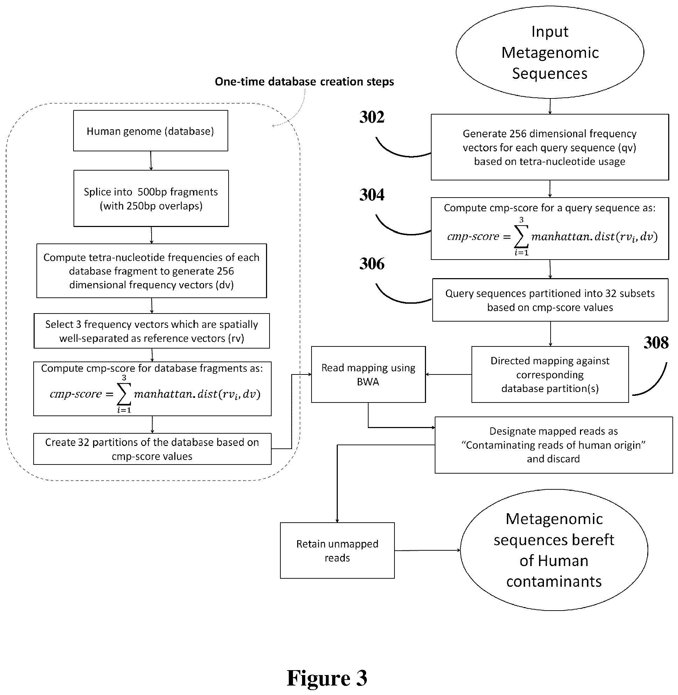

Referring to Figure 3 is a flow chart illustrating a method for representing compositional properties of a biological sequence fragment in an embodiment that exemplifies an application of the depicted method in the field of metagenomics.

In the present invention, the unidimensional compositional metric (cmp-score) is utilized for identifying the subset of DNA fragments of human origin which contaminate human-host derived metagenomic datasets.

Utilization of cmp-score for identification and subsequent removal of human-origin reads in metagenomic data sets, is based on the following premise. Sequence similarity between two DNA sequences in most cases translates to approximate similarity in their compositional characteristics. Consequently, instead of searching and mapping all query sequences from a given metagenomic dataset, en masse to the entire human genome, it would be beneficial in terms of both time and memory, if the query sequences can be first either categorized, sorted or ordered according to their compositional features, and subsequently searched or mapped only against the subset of human genome fragments having similar compositional features. Efficiency of the directed-mapping strategy depends on the metric that defines compositional similarity. The cmp-score metric is utilized for this purpose in the current implementation.

At the step 302, the 256 dimensional tetra-nucleotide frequency vectors are generated for all 'query' sequences constituting the metagenomic dataset. Computing the cmp-score for any given DNA fragment, involves comparing the tetra-nucleotide frequency vector corresponding to the fragment with three or more reference points or reference vectors in the 256 dimensional feature vector space. For the purpose of the present implementation, 'three reference vectors' were chosen using the following procedure. In the current implementation, DNA sequence fragments of length 500 base pairs (bp), each were randomly generated from the entire human genome. For each of these sequence fragments, vectors representing the frequencies of all possible tetra-nucleotides in that sequence were computed. Guided by principal component analysis (PCA), and following the steps for generating a set of reference vectors as described earlier, three spatially well separated vectors were then chosen as the reference vectors henceforth referred to as rv1, rv2 and rv3.

In the present implementation, the spatially well separated vectors were generated by taking DNA fragments from the database i.e. human genome. In other implementation based on the end objectives or requirements, these spatially well separated vectors may be generated from DNA sequence fragments constituting the query dataset itself and/or obtained using mathematical procedures and/or DNA sequence fragments of a predetermined length are randomly generated from completely sequenced/ draft sequenced genomes from any other data source. It should be noted that the length of the randomly generated DNA sequence fragments may be determined based on the average length of query sequence(s) for which cmp-score needs to be generated.

At the step 304, cmp-scores are computed. In the present implementation, the cmp-score for any given DNA sequence was subsequently calculated as the cumulative Manhattan distance between its tetra-nucleotide frequency vector (v) and each of the 'three' reference vectors (rv1, rv2 and rv3) generated in step 1 described above.

$$cmp\text{-}score = dist(v,rv1) + dist(v,rv2) + dist(v,rv3)$$

In the present implementation, the cmp-score was generated based on Manhattan distance. In other implementations, other distance measures such as Euclidean or Chebyshev etc. may be employed. In the present implementation, the cmp-score was computed based on 3 reference vectors. In other implementations, more than 3 reference vectors may be employed.

Following a set of one time database creation steps, the human genome database is partitioned into smaller subsets based on cmp-scores. The human genome was partitioned into compositionally similar subsets, each set containing fragments having *cmp-score* values in a pre-defined range. In order to create these subsets, the human chromosomal sequences were first segmented into 500 bp fragments with an overlap of 250 bp. The *cmp-score* values were computed for each of these fragments as described in step 304. The majority of the *cmp-score* values were observed to range between 900-1525. In the present implementation, based on the *cmp-score* values, the human DNA fragments were partitioned into 32 subsets. These subsets correspond to the following pre-defined *cmp-score* ranges -
<910, 911-930, 931-950, 951-970, 971-990, 991-1010, 1011-1030, 1031-1050, 1051-1070, 1071-1090, 1091-1110, 1111-1130, 1131-1150, 1151-1170, 1171-1190, 1191-1210, 1211-1230, 1231-1250, 1251-1270, 1271-1290, 1291-1310, 1311-1330, 1331-1350, 1351-1370, 1371-1390, 1391-1410, 1411-1430, 1431-1450, 1451-1470, 1471-1490, 1491-1510, >1510

Sequence fragments in each subset were appropriately formatted and subsequently indexed using the BWA algorithm. This partitioned human genome database is used by the cmp-score workflow for the directed read mapping step 308.

At the step 306, the query sequences constituting the metagenomic dataset is partitioned into 32 subsets, based on cmp-score, to be used for the directed read-mapping. For the directed read-mapping step, *cmp-score* values for each of the query sequences, brought forward from the first step, are computed as mentioned in step 304. Based on the *cmp-score* values, the query sequences are sorted and partitioned into 32 sub-groups, having *cmp-score* ranges identical to those of the (human) database partitions.

At step 308, sequences in each of the 32 query sequence sub-groups are then mapped, using the fastmap application of BWA, to appropriate subsets of the pre-partitioned human genome database. For directed mapping of sequences belonging to each query sub-group, specific subsets of the partitioned human genome database are considered. These subsets are chosen such that their *cmp-score* values lie in the range of +/-60 with respect to those of the query sub-group. The range of '+/-60' was determined empirically by calculating *cmp-score* values of a large number of randomly generated human genome fragments, and comparing these *cmp-score* values against those of their closest counterparts (similar sequences) in the pre-partitioned human genome database.

The fastmap application of BWA is designed for mapping or aligning sequences without any gaps or substitutions. The results obtained from the fastmap tool are parsed by the cmp-score algorithm and 'stitched' together into longer alignments. This allows accommodation for natural variations in the human genome as well as sequencing errors. Query sequences from a metagenomic dataset, which align to the fragments in the pre-partitioned human genome database with >=96% identity, are categorized as human genome contaminants. These contaminant sequences are removed from the query metagenomic dataset to obtain an output file which is bereft of contaminating human genome sequences.

Further, cmp-score based human contamination removal procedure is validated with simulated metagenomic datasets. A total of 18 simulated metagenomic datasets were used for validating the performance of cmp-score based contamination removal procedure. While 80% of reads in each dataset originated from prokaryotic genomes, randomly pooled from completely sequenced prokaryotic genomes available in the NCBI database, the remaining 20% were sourced from the human genome. Based on the length of constituent reads, the 18 datasets were divided into three equal groups, of average read-lengths around 250 bp, 400 bp, and 600 bp. These read-lengths are representative of present day sequencing technologies such as Illumina-MiSeq, Roche-454 which are routinely employed in metagenomic sequencing studies. While the sequence length of paired-end reads (150bp x 2) from Illumina is in the minimum range of 250-300 bp, when merged, different Roche-454 sequencing platforms yield sequences having average lengths of 250, 400 and 600 bp. Based on the number of reads, 1 million, 2.5 million and 5 million in each dataset, each group was further subdivided into 3 subgroups, having 2 datasets each. Given that the present generation of sequencing technologies are reported to have a sequencing error rate of around 1%, in-house scripts were employed for introducing 1% random mutations including insertions, deletions, substitutions in one of the datasets in each subgroup. For the purpose of comparison, all datasets were individually analyzed using cmp-score-based contamination removal procedure as well as a state-of-the-art program meant for the same purpose i.e. DeconSeq. The parameters of DeconSeq were suitably modified to enable it to identify human sequences (with an allowed error rate of 1%). Results were analysed with respect to (a) total execution time, (b) peak memory usage, and (c) sensitivity and specificity of detecting contaminating human sequences. For each individual dataset, the peak memory requirements for both cmp-score-based contamination removal procedure and DeconSeq were also captured. All validation experiments were performed on a system with an Intel Xeon processor (2.33 GHz) with 64GB RAM.

Following tables summarizes the results:

Table 1: This table indicates the ability of cmp-score-based contamination removal procedure in terms of sensitivity and specificity of detecting contaminating human sequences

| Dataset | Length of sequences (bp) | Percentage of mutations | No of sequences in dataset | | Total Number of sequences in dataset | Sensitivity of detecting human sequences | Specificity of detecting human sequences |
|---|---|---|---|---|---|---|---|
| | | | Prokaryotic | Human | | | |
| PH_250_1M_0mut.ffn | 250 | 0 | 800000 | 200000 | 1000000 | 0.99 | 0.97 |
| PH_250_1M_1mut.ffn | 250 | 1 | 800000 | 200000 | 1000000 | 0.98 | 0.97 |
| | | | | | | | |
| PH_250_2.5M_0mut.ffn | 250 | 0 | 2000000 | 500000 | 2500000 | 0.99 | 0.97 |
| PH_250_25M_1mut.ffn | 250 | 1 | 2000000 | 500000 | 2500000 | 0.99 | 0.97 |
| | | | | | | | |
| PH_250_5M_0mut.ffn | 250 | 0 | 4000000 | 1000000 | 5000000 | 0.99 | 0.97 |
| PH_250_5M_1mut.ffn | 250 | 1 | 4000000 | 1000000 | 5000000 | 0.99 | 0.97 |
| | | | | | | | |
| PH_400_1M_0mut.ffn | 400 | 0 | 800000 | 200000 | 1000000 | 0.99 | 0.99 |
| PH_400_1M_1mut.ffn | 400 | 1 | 800000 | 200000 | 1000000 | 0.98 | 0.99 |
| | | | | | | | |

| Dataset | Length of sequences (bp) | Percentage of mutations | No of sequences in dataset | | Total Number of sequences in dataset | Sensitivity of detecting human sequences | Specificity of detecting human sequences |
|---|---|---|---|---|---|---|---|
| | | | Prokaryotic | Human | | | |
| PH_ 400_2.5M_ 0mut.ffn | 400 | 0 | 2000000 | 500000 | 2500000 | 0.99 | 0.99 |
| PH_ 400_2.5M_ 1mut.ffn | 400 | 1 | 2000000 | 500000 | 2500000 | 0.98 | 0.99 |
| | | | | | | | |
| PH_ 400_5M_ 0mut.ffn | 400 | 0 | 4000000 | 1000000 | 5000000 | 0.99 | 0.99 |
| PH_ 400_5M_ 1mut.ffn | 400 | 1 | 4000000 | 1000000 | 5000000 | 0.98 | 0.99 |
| | | | | | | | |
| PH_ 600_1M_ 0mut.ffn | 600 | 0 | 800000 | 200000 | 1000000 | 0.99 | 0.99 |
| PH_ 600_1M_ 1mut.ffn | 600 | 1 | 800000 | 200000 | 1000000 | 0.99 | 0.99 |
| | | | | | | | |
| PH_ 600_2.5M_ 0mut.ffn | 600 | 0 | 2000000 | 500000 | 2500000 | 0.99 | 0.99 |
| PH_ 600_2.5M_ 1mut.ffn | 600 | 1 | 2000000 | 500000 | 2500000 | 0.99 | 0.99 |

(continued)

| Dataset | Length of sequences (bp) | Percentage of mutations | No of sequences in dataset | | Total Number of sequences in dataset | Sensitivity of detecting human sequences | Specificity of detecting human sequences |
|---|---|---|---|---|---|---|---|
| | | | Prokaryotic | Human | | | |
| PH_600_5M_0mut.ffn | 600 | 0 | 4000000 | 1000000 | 5000000 | 0.99 | 0.99 |
| PH_600_5M_1mut.ffn | 600 | 1 | 4000000 | 1000000 | 5000000 | 0.99 | 0.99 |

Table 2: This table provides a comparison of total execution time and peak memory usage statistics for detecting contaminating sequences using an implementation employing cmp-scores, and DeConseq

| Input Dataset | Peak memory usage for detecting contaminating sequences (in Gigabytes) | | Time taken for detecting contaminating sequences (in Minutes) | |
|---|---|---|---|---|
| | Current method utilizing cmp-scores | Using DeConseq (state of art) | Current method utilizing cmp-scores | Using DeConseq (state of art) |
| 1M (250 bp) | 1.8 | 4.5 | 33 | 39 |
| 1M (400 bp) | 1.9 | 5.2 | 39 | 65 |
| 1M (600 bp) | 2.1 | 6.2 | 36 | 106 |
| 2.5M (250 bp) | 1.9 | 6.3 | 80 | 96 |
| 2.5M (400 bp) | 2.1 | 8.1 | 89 | 163 |
| 2.5M (600 bp) | 2.2 | 10.5 | 93 | 255 |
| 5M (250 bp) | 2 | 9.3 | 179 | 193 |
| 5M (400 bp) | 2.1 | 12.9 | 176 | 326 |
| 5M (600 bp) | 2.3 | 17.6 | 185 | 517 |

The present invention provides the method and system for representing compositional properties of a biological sequence fragment using the unidimensional compositional metric. Further, the method and system may be appropriately modified and extended to non-nucleotide biological sequences such as amino-acid sequences.

The present invention represents biological sequences using a unidimensional compositional metric. The unidimensional compositional metric used in the present invention is able to sufficiently capture the compositional features of any query sequence. The present invention therefore proposes an efficient way of scaling multidimensional biological sequence composition vectors to a unidimensional metric. The unidimensional compositional metric has applicability in downstream bioinformatics applications which involve large-scale comparison of biological sequences. The unidimensional compositional metric, being unidimensional, enables rapid comparison and segregation of biological sequences, and computations using this metric are significantly less compute intensive.

## Claims

1. A method for representing compositional properties of each of the biological sequence fragments using a unidimensional compositional metric, wherein the method is **characterized in** generating a set of spatially well separated reference vectors in a feature vector space pertaining to said compositional properties of the biological sequence fragments, for generating unidimensional compositional metric; wherein for representing compositional properties of each of the biological sequence fragments using the unidimensional compositional metric, the method comprises processor implemented steps of:

    collecting a plurality of biological sequence fragments using a biological sequence fragment collection module (202), wherein the plurality of biological sequences is collected from a group consisting of genomic, metagenomic or environmental samples;
    sequencing the plurality of biological sequence fragments using a biological sequence fragment sequencing module (204);

generating a 256-dimensional tetra-nucleotide frequency vector (v) corresponding to each sequenced biological sequence fragment out of the plurality of sequenced biological sequence fragments;

subjecting the 256-dimensional tetra-nucleotide frequency vectors to Principal Component Analysis (PCA);

selecting a pair of discrete vectors, wherein each of the pair of the discrete vectors lies at extremes of a first principal component (PC1) and are maximally separated along PC1;

repeating selecting of the pair of discrete vectors for each of PC2, PC3, ..., PCn, to select two discrete vectors in each iteration for generating a first set of reference vectors using a reference vectors generation module (206), wherein the first set of reference vectors comprises of the pairs of discrete vectors arranged in an order of their selection, wherein the order comprises the pair of discrete vectors derived from the extremes of the most significant principal components preceding the pair of discrete vectors derived from the extremes of relatively less significant principal components;

computing the unidimensional compositional metric for each sequenced biological sequence fragment out of the plurality of sequenced biological sequence fragments as a cumulative function of a distance of the 256 - dimensional tetra-nucleotide frequency vector (v) corresponding to an individual biological sequence fragment from first three or more reference vectors selected out of the first set of reference vectors using a unidimensional compositional metric computation module (208);

segregating each sequenced biological sequence fragment out of the plurality of sequenced biological sequence fragments into a plurality of groups based on respective value of the unidimensional compositional metric using a sequenced biological sequence fragment segregation module (210); and

utilizing the segregated plurality of groups of the sequenced biological fragments for identifying one or more metagenomic sequences bereft of human contaminants, wherein identifying the one or more metagenomic sequences bereft of human contaminants comprises:

> sorting and partitioning the sequences into sub-groups having the unidimensional compositional metric ranges identical to ranges of human genome database partitions;
>
> mapping sequences in each of the sequence sub-groups to appropriate subsets of the partitioned human genome database;
>
> comparing the unidimensional compositional metric values against similar sequences in the pre-partitioned human genome database to identify the sequences from a metagenomic dataset, which align to the fragments in the pre-partitioned human genome database with a predefined value of identity;
>
> categorizing the aligned fragments as human genome contaminants; and removing identified contaminant sequences fragments from the plurality of sequences fragments.

2. The method according to claim 1, wherein the unidimensional compositional metric is cmp-score, wherein the cmp-score is calculated as a cumulative distance between the 256-dimensional tetra-nucleotide frequency vector (v) corresponding to the individual biological sequence fragment and each of the three or more reference vectors (rv1, rv2, rv3, .....rvN) using following equation cmp-score = dist (v-rv1) + dist (v-rv2) + dist (v-rv3) + ... + dist (v-rvN), and wherein the cmp-score is utilized for identifying subset of DNA fragments of human origin contaminating human-host derived metagenomic datasets.

3. The method according to claim 1, wherein the distance between the 256-dimensional tetra-nucleotide frequency vector (v) corresponding to each sequenced biological sequence fragment out of the plurality of sequenced biological sequence fragments is computed using a distance metric selected from a group comprising Manhattan distance, Euclidean distance, and an appropriate metric suitable for measuring distance in a multidimensional space.

4. The method according to claim 1, further comprises of generating n-dimensional frequency vector for a plurality of k-mer frequencies wherein the plurality of k-mer frequencies are other than tetra-nucleotide frequency.

5. The method according to claim 1, wherein the reference vectors constitutes randomly generated 256 dimensional vectors that are discrete in feature vector space.

6. The method according to claim 1, further comprises of computing the unidimensional compositional metric for each sequenced biological sequence fragment out of the plurality of sequenced biological sequence fragments as a cumulative function of the distance of the tetra-nucleotide frequency vector (v) corresponding to an individual biological sequence fragment from the first three or more reference vectors, wherein the three or more reference vectors are derived from a second set of reference vectors.

7. The method according to claim 6, wherein derivation of the second set of reference vectors comprising steps of generating a 256-dimensional tetra-nucleotide frequency vector (v) corresponding to a plurality of randomly generated biological sequence fragments of a predetermined length, subjecting the 256-dimensional tetra-nucleotide frequency vectors to Principal Component Analysis (PCA); selecting two vectors that lie at the extremes of the first principal component (PC1) and are therefore maximally separated along PC1; repeating the selection of two discrete vectors for each of PC2, PC3, ..., PCn, so as to select two discrete vectors in each iteration for generating the second set of reference vectors wherein the second set of reference vectors comprises of the discrete vector pairs arranged in the order of their selection, in an order in which the reference vector pairs derived from the extremes of the most significant principal components precede reference vector pairs derived from the extremes of relatively less significant principal components.

8. The method according to claim 6, wherein the plurality of randomly generated biological sequence fragments are derived from completely sequenced genomes.

9. The method according to claim 1, wherein generating the 256-dimensional tetra-nucleotide frequency vector (v) corresponding to the each sequenced biological sequence fragment out of the plurality of sequenced biological sequence fragments; subjecting the 256-dimensional tetra-nucleotide frequency vectors to Principal Component Analysis (PCA); selecting two vectors that lie at the extremes of the first principal component (PC1) and are therefore maximally separated along PC1; repeating the selection of two discrete vectors for each of PC2, PC3, ..., PCn, so as to select two discrete vectors in each iteration for generating the first set of reference vectors using the reference vectors generation module (206) wherein the first set of reference vectors comprises of the discrete vector pairs arranged in the order of their selection, in the order in which the reference vector pairs derived from the extremes of the most significant principal components precede reference vector pairs derived from the extremes of relatively less significant principal components, is a one-time process.

10. A system (200) for representing compositional properties of each of the biological sequence fragments using a unidimensional compositional metric, wherein the method is **characterized in** generating a set of spatially well separated reference vectors in a feature vector space pertaining to said compositional properties of the biological sequence fragments, for generating said unidimensional metric; wherein for representing composition properties of each of the biological sequence fragments using the unidimensional compositional metric, the system (200) comprises:

a. a processor;
b. a data bus coupled to said processor;
c. a computer-usable medium embodying computer code, said computer-usable medium being coupled to said data bus, said computer program code comprising instructions executable by said processor and configured for executing:

a biological sequence fragment collection module (202) adapted for collecting a plurality of biological sequence fragments, wherein the plurality of biological sequences is collected from a group consisting of genomic, metagenomic or environmental samples;
a biological sequence fragment sequencing module (204) adapted for sequencing the plurality of biological sequence fragments;
a reference vectors generation module (206) adapted for generating a 256-dimensional tetra-nucleotide frequency vector (v) corresponding to each sequenced biological sequence fragment out of the plurality of sequenced biological sequence fragments; subjecting the 256-dimensional tetra-nucleotide frequency vectors to Principal Component Analysis (PCA); selecting a pair of discrete vectors, wherein the pair of the discrete vectors lie at extremes of a first principal component (PC1) and are maximally separated along PC1; repeating the selecting of the pair of discrete vectors for each of PC2, PC3, ..., PCn, to select two discrete vectors in each iteration for generating a first set of reference vectors, wherein the first set of reference vectors comprises of the pairs of discrete vectors arranged in an order of their selection, wherein the order comprises the pair of discrete vectors derived from the extremes of a most significant principal components preceding the pair of discrete vectors derived from the extremes of relatively less significant principal components;
a unidimensional compositional metric computation module (208) adapted for computing the unidimensional compositional metric for each sequenced biological sequence fragment out of the plurality of sequenced biological sequence fragments as a cumulative function of a distance of the 256-dimensional tetra-nucleotide frequency vector (v) corresponding to an individual biological sequence fragment from first three or more reference vectors selected out of the first set of reference vectors; and

a sequenced biological sequence fragment segregation module (210) adapted for segregating each sequenced biological sequence fragment out of the plurality of sequenced biological sequence fragments into a plurality of groups based on respective value of the unidimensional compositional metric, wherein the system (200) is adapted utilizing the segregated plurality of groups of the sequenced biological fragments for identifying one or more metagenomic sequences bereft of human contaminants, wherein identifying the one or more metagenomic sequences bereft of human contaminants comprises:

sorting and partitioning the sequences into sub-groups having the unidimensional compositional metric ranges identical to ranges of human genome database partitions;

mapping sequences in each of the sequence sub-groups to appropriate subsets of the partitioned human genome database;

comparing the unidimensional compositional metric values against similar sequences in the pre-partitioned human genome database to identify the sequences from a metagenomic dataset, which align to the fragments in the pre-partitioned human genome database with a predefined value of identity;

categorizing the aligned fragments as human genome contaminants; and removing identified contaminant sequences fragments from the plurality of sequences fragments.

11. A non-transitory computer-readable medium having embodied thereon a computer program for representing compositional properties of each of the biological sequence fragments using a unidimensional compositional metric, **characterized in** generating a set of spatially well separated reference vectors in a feature vector space pertaining to said compositional properties of the biological sequence fragment, for generating unidimensional compositional metric; wherein for representing compositional properties of each biological sequence fragment using the unidimensional compositional metric, the computer program is to implement processor implemented steps of:

collecting a plurality of biological sequence fragments using a biological sequence fragment collection module (202), wherein the plurality of biological sequences is collected from a group consisting of genomic, metagenomic or environmental samples;

sequencing the plurality of biological sequence fragments using a biological sequence fragment sequencing module (204);

generating a 256-dimensional tetra-nucleotide frequency vector (v) corresponding to each sequenced biological sequence fragment out of the plurality of sequenced biological sequence fragments;

subjecting the 256-dimensional tetra-nucleotide frequency vectors to Principal Component Analysis (PCA);

selecting a pair of discrete vectors, wherein each of the pair of discrete vectors lies at extremes of a first principal component (PC1) and are t maximally separated along PC 1;

repeating the selecting of the pair of discrete vectors for each of PC2, PC3, ..., PCn, to select two discrete vectors in each iteration for generating a first set of reference vectors using a reference vectors generation module (206) wherein the first set of reference vectors comprises of the pairs of discrete vectors arranged in an order of their selection, wherein the order comprises the pair of discrete vectors derived from the extremes of a most significant principal components preceding the pair of discrete vectors derived from the extremes of relatively less significant principal components;

computing the unidimensional compositional metric for each sequenced biological sequence fragment out of the plurality of sequenced biological sequence fragments as a cumulative function of a distance of the 256-dimensional tetra-nucleotide frequency vector (v) corresponding to an individual biological sequence fragment from the first three or more reference vectors selected out of the first set of reference vectors using a unidimensional compositional metric computation module (208);

segregating each sequenced biological sequence fragment out of the plurality of sequenced biological sequence fragments into a plurality of groups based on respective value of the unidimensional compositional metric using a sequenced biological sequence fragment segregation module (210); and

utilizing the segregated plurality of groups of the sequenced biological fragments for identifying one or more metagenomic sequences bereft of human contaminants, wherein identifying the one or more metagenomic sequences bereft of human contaminants comprises:

sorting and partitioning the sequences into sub-groups having the unidimensional compositional metric ranges identical to ranges of human genome database partitions;

mapping sequences in each of the sequence sub-groups to appropriate subsets of the partitioned human genome database;

comparing the unidimensional compositional metric values against similar sequences in the pre-partitioned human genome database to identify the sequences from a metagenomic dataset, which align to the fragments in the pre-partitioned human genome database with a predefined value of identity;
categorizing the aligned fragments as human genome contaminants; and removing identified contaminant sequences fragments from the plurality of sequences fragments.

**Patentansprüche**

1. Verfahren zum Darstellen von Zusammensetzungseigenschaften von jedem der biologischen Sequenzfragmente unter Verwendung einer eindimensionalen Zusammensetzungsmetrik, wobei das Verfahren **gekennzeichnet ist durch** Erzeugen eines Satzes von räumlich gut getrennten Referenzvektoren in einem Merkmalsvektorraum, der zu den Zusammensetzungseigenschaften der biologischen Sequenzfragmente gehört, zum Erzeugen einer eindimensionalen Zusammensetzungsmetrik; wobei zum Darstellen von Zusammensetzungseigenschaften von jedem der biologischen Sequenzfragmente unter Verwendung der eindimensionalen Zusammensetzungsmetrik das Verfahren prozessorimplementierte Schritte umfasst zum:

Sammeln einer Mehrzahl von biologischen Sequenzfragmenten unter Verwendung eines Sammelmoduls (202) für biologische Sequenzfragmente, wobei die Mehrzahl von biologischen Sequenzen aus einer Gruppe bestehend aus genomischen, metagenomischen oder Umweltproben gesammelt wird;
Sequenzieren der Mehrzahl von biologischen Sequenzfragmenten unter Verwendung eines Sequenziermoduls (204) für biologische Sequenzfragmente;
Erzeugen eines 256-dimensionalen Tetranukleotidfrequenzvektors (v), der jedem sequenzierten biologischen Sequenzfragment aus der Mehrzahl von sequenzierten biologischen Sequenzfragmenten entspricht;
Unterziehen der 256-dimensionalen Tetranukleotidfrequenzvektoren einer Hauptkomponentenanalyse (PCA);
Auswählen eines Paares von diskreten Vektoren, wobei jeder des Paares der diskreten Vektoren an Extrema einer ersten Hauptkomponente (PC 1) liegt und entlang PC1 maximal getrennt ist;
Wiederholen des Auswählens des Paares von diskreten Vektoren für jeden von PC2, PC3, ..., PCn, um in jeder Iteration zwei diskrete Vektoren zum Erzeugen eines ersten Satzes von Referenzvektoren unter Verwendung eines Referenzvektorerzeugungsmoduls (206) auszuwählen, wobei der erste Satz von Referenzvektoren Paare von diskreten Vektoren umfasst, die in einer Reihenfolge ihrer Auswahl angeordnet sind, wobei die Reihenfolge das Paar von diskreten Vektoren umfasst, das von den Extrema der wichtigsten Hauptkomponenten abgeleitet ist, die dem Paar von diskreten Vektoren vorausgehen, die von den Extrema von relativ weniger wichtigen Hauptkomponenten abgeleitet sind;
Berechnen der eindimensionalen Zusammensetzungsmetrik für jedes sequenzierte biologische Sequenzfragment aus der Mehrzahl von sequenzierten biologischen Sequenzfragmenten als eine kumulative Funktion eines Abstands des 256-dimensionalen Tetranukleotidfrequenzvektors (v), der einem individuellen biologischen Sequenzfragment aus den ersten drei oder mehr Referenzvektoren entspricht, die aus dem ersten Satz von Referenzvektoren ausgewählt sind, unter Verwendung eines Berechnungsmoduls (208) für eine eindimensionale Zusammensetzungsmetrik;
Trennen jedes sequenzierten biologischen Sequenzfragments aus der Mehrzahl von sequenzierten biologischen Sequenzfragmenten in eine Mehrzahl von Gruppen basierend auf einem jeweiligen Wert der eindimensionalen Zusammensetzungsmetrik unter Verwendung eines Trennmoduls (210) für sequenzierte biologische Sequenzfragmente; und

Verwenden der getrennten Mehrzahl von Gruppen der sequenzierten biologischen Fragmente zum Identifizieren einer oder mehrerer metagenomischer Sequenzen, die von menschlichen Verunreinigungen betroffen sind, wobei das Identifizieren der einen oder mehreren metagenomischen Sequenzen, die von menschlichen Verunreinigungen betroffen sind, umfasst:

Sortieren und Partitionieren der Sequenzen in Untergruppen, die die eindimensionalen Zusammensetzungsmetrikbereiche aufweisen, die mit Bereichen von menschlichen Genomdatenbankpartitionen identisch sind;
Zuordnen von Sequenzen in jeder der Sequenzuntergruppen zu geeigneten Untergruppen der partitionierten menschlichen Genomdatenbank;
Vergleichen der eindimensionalen Zusammensetzungsmetrikwerte mit ähnlichen Sequenzen in der vorpartitionierten menschlichen Genomdatenbank, um die Sequenzen aus einem metagenomischen Datensatz zu identifizieren, der sich mit den Fragmenten in der vorpartitionierten menschlichen Genomdatenbank mit einem vordefinierten Identitätswert ausrichtet;
Kategorisieren der ausgerichteten Fragmente als menschliche Genomverunreinigungen; und Entfernen identifizierter Verunreinigungssequenzfragmente aus der Mehrzahl von Sequenzfragmenten.

2. Verfahren nach Anspruch 1, wobei die eindimensionale Zusammensetzungsmetrik cmp-Score ist, wobei der cmp-Score als ein kumulativer Abstand zwischen dem 256-dimensionalen Tetranukleotidfrequenzvektor (v), der dem individuellen biologischen Sequenzfragment entspricht, und jedem der drei oder mehr Referenzvektoren (rv1, rv2, rv3, ..., rvN) unter Verwendung der folgenden Gleichung berechnet wird: cmp-Score = dist (v-rv1) + dist (v-rv2) + dist (v-rv3) + ... + dist (v-rvN), und wobei der cmp-Score zum Identifizieren einer Untergruppe von DNA-Fragmenten menschlichen Ursprungs verwendet wird, die von Menschen-Wirt abgeleitete metagenomische Datensätze verunreinigen.

3. Verfahren nach Anspruch 1, wobei der Abstand zwischen dem 256-dimensionalen Tetranukleotidfrequenzvektor (v), der jedem sequenzierten biologischen Sequenzfragment aus der Mehrzahl von sequenzierten biologischen Sequenzfragmenten entspricht, unter Verwendung einer Abstandsmetrik berechnet wird, die aus einer Gruppe ausgewählt ist, die Manhattan-Abstand, euklidischen Abstand und eine geeignete Metrik umfasst, die zum Messen von Abstand in einem mehrdimensionalen Raum geeignet ist.

4. Verfahren nach Anspruch 1, das ferner das Erzeugen eines n-dimensionalen Frequenzvektors für eine Mehrzahl von k-Mer-Frequenzen umfasst, wobei die Mehrzahl von k-Mer-Frequenzen von der Tetranukleotidfrequenz verschieden ist.

5. Verfahren nach Anspruch 1, wobei die Referenzvektoren zufällig erzeugte 256-dimensionale Vektoren bilden, die im Merkmalsvektorraum diskret sind.

6. Verfahren nach Anspruch 1, das ferner das Berechnen der eindimensionalen Zusammensetzungsmetrik für jedes sequenzierte biologische Sequenzfragment aus der Mehrzahl von sequenzierten biologischen Sequenzfragmenten als eine kumulative Funktion des Abstands des Tetranukleotidfrequenzvektors (v), der einem individuellen biologischen Sequenzfragment aus den ersten drei oder mehr Referenzvektoren entspricht, umfasst, wobei die drei oder mehr Referenzvektoren aus einem zweiten Satz von Referenzvektoren abgeleitet sind.

7. Verfahren nach Anspruch 6, wobei die Ableitung des zweiten Satzes von Referenzvektoren Schritte umfasst zum Erzeugen eines 256-dimensionalen Tetranukleotidfrequenzvektors (v), der einer Mehrzahl von zufällig erzeugten biologischen Sequenzfragmenten einer vorbestimmten Länge entspricht, Unterziehen der 256-dimensionalen Tetranukleotidfrequenzvektoren einer Hauptkomponentenanalyse (PCA); Auswählen von zwei Vektoren, die an den Extrema der ersten Hauptkomponente (PC1) liegen und daher entlang PC1 maximal getrennt sind; Wiederholen der Auswahl von zwei diskreten Vektoren für jeden von PC2, PC3, ..., PCn, um in jeder Iteration zwei diskrete Vektoren zum Erzeugen des zweiten Satzes von Referenzvektoren auszuwählen, wobei der zweite Satz von Referenzvektoren Paare von diskreten Vektoren umfasst, die in der Reihenfolge ihrer Auswahl angeordnet sind, in einer Reihenfolge, in der die Referenzvektorpaare, die von den Extrema der wichtigsten Hauptkomponenten abgeleitet sind, Referenzvektorpaaren vorausgehen, die von den Extrema von relativ weniger wichtigen Hauptkomponenten abgeleitet sind.

8. Verfahren nach Anspruch 6, wobei die Mehrzahl von zufällig erzeugten biologischen Sequenzfragmenten von vollständig sequenzierten Genomen abgeleitet ist.

9. Verfahren nach Anspruch 1, wobei das Erzeugen des 256-dimensionalen Tetranukleotidfrequenzvektors (v), der jedem sequenzierten biologischen Sequenzfragment aus der Mehrzahl von sequenzierten biologischen Sequenzfragmenten entspricht; Unterziehen der 256-dimensionalen Tetranukleotidfrequenzvektoren einer Hauptkomponentenanalyse (PCA); Auswählen von zwei Vektoren, die an den Extrema der ersten Hauptkomponente (PC1) liegen und daher entlang PC1 maximal getrennt sind; Wiederholen der Auswahl von zwei diskreten Vektoren für jeden von PC2, PC3, ..., PCn, um in jeder Iteration zwei diskrete Vektoren zum Erzeugen des ersten Satzes von Referenzvektoren unter Verwendung des Referenzvektorerzeugungsmoduls (206) auszuwählen, wobei der erste Satz von Referenzvektoren Paare von diskreten Vektoren umfasst, die in der Reihenfolge ihrer Auswahl angeordnet sind, in der Reihenfolge, in der die Referenzvektorpaare, die von den Extrema der wichtigsten Hauptkomponenten abgeleitet sind, Referenzvektorpaaren vorausgehen, die von den Extrema von relativ weniger wichtigen Hauptkomponenten abgeleitet sind, ein einmaliger Prozess ist.

10. System (200) zum Darstellen von Zusammensetzungseigenschaften von jedem der biologischen Sequenzfragmente unter Verwendung einer eindimensionalen Zusammensetzungsmetrik, wobei das Verfahren **gekennzeichnet ist**

**durch** Erzeugen eines Satzes von räumlich gut getrennten Referenzvektoren in einem Merkmalsvektorraum, der zu den Zusammensetzungseigenschaften der biologischen Sequenzfragmente gehört, zum Erzeugen der eindimensionalen Metrik; wobei zum Darstellen von Zusammensetzungseigenschaften von jedem der biologischen Sequenzfragmente unter Verwendung der eindimensionalen Zusammensetzungsmetrik das System (200) umfasst:

a. einen Prozessor;
b. einen Datenbus, der mit dem Prozessor gekoppelt ist;
c. ein computerverwendbares Medium, das Computercode verkörpert, wobei das computerverwendbare Medium mit dem Datenbus gekoppelt ist, wobei der Computerprogrammcode Anweisungen umfasst, die durch den Prozessor ausführbar sind und konfiguriert sind zum Ausführen:

eines Sammelmoduls (202) für biologische Sequenzfragmente, das zum Sammeln einer Mehrzahl von biologischen Sequenzfragmenten ausgelegt ist, wobei die Mehrzahl von biologischen Sequenzen aus einer Gruppe bestehend aus genomischen, metagenomischen oder Umweltproben gesammelt wird;
eines Sequenziermoduls (204) für biologische Sequenzfragmente, das zum Sequenzieren der Mehrzahl von biologischen Sequenzfragmenten ausgelegt ist;
eines Referenzvektorerzeugungsmoduls (206), das zum Erzeugen eines 256-dimensionalen Tetranukleotidfrequenzvektors (v) ausgelegt ist, der jedem sequenzierten biologischen Sequenzfragment aus der Mehrzahl von sequenzierten biologischen Sequenzfragmenten entspricht; Unterziehen der 256-dimensionalen Tetranukleotidfrequenzvektoren einer Hauptkomponentenanalyse (PCA); Auswählen eines Paares von diskreten Vektoren, wobei das Paar der diskreten Vektoren an Extrema einer ersten Hauptkomponente (PC1) liegt und entlang PC1 maximal getrennt ist; Wiederholen des Auswählens des Paares von diskreten Vektoren für jeden von PC2, PC3, ..., PCn, um in jeder Iteration zwei diskrete Vektoren zum Erzeugen eines ersten Satzes von Referenzvektoren auszuwählen, wobei der erste Satz von Referenzvektoren Paare von diskreten Vektoren umfasst, die in einer Reihenfolge ihrer Auswahl angeordnet sind, wobei die Reihenfolge das Paar von diskreten Vektoren umfasst, das von den Extrema einer wichtigsten Hauptkomponente abgeleitet ist, die dem Paar von diskreten Vektoren vorausgeht, die von den Extrema von relativ weniger wichtigen Hauptkomponenten abgeleitet sind;
eines Berechnungsmoduls (208) für eine eindimensionale Zusammensetzungsmetrik, das zum Berechnen der eindimensionalen Zusammensetzungsmetrik für jedes sequenzierte biologische Sequenzfragment aus der Mehrzahl von sequenzierten biologischen Sequenzfragmenten als eine kumulative Funktion eines Abstands des 256-dimensionalen Tetranukleotidfrequenzvektors (v) ausgelegt ist, der einem individuellen biologischen Sequenzfragment aus den ersten drei oder mehr Referenzvektoren entspricht, die aus dem ersten Satz von Referenzvektoren ausgewählt sind; und

eines Trennmoduls (210) für sequenzierte biologische Sequenzfragmente, das zum Trennen jedes sequenzierten biologischen Sequenzfragments aus der Mehrzahl von sequenzierten biologischen Sequenzfragmenten in eine Mehrzahl von Gruppen basierend auf einem jeweiligen Wert der eindimensionalen Zusammensetzungsmetrik ausgelegt ist, wobei das System (200) zum Verwenden der getrennten Mehrzahl von Gruppen sequenzierten biologischen Fragmente zum Identifizieren einer oder mehrerer metagenomischer Sequenzen ausgelegt ist, die von menschlichen Verunreinigungen betroffen sind, wobei das Identifizieren der einen oder mehreren metagenomischen Sequenzen, die von menschlichen Verunreinigungen betroffen sind, umfasst:

Sortieren und Partitionieren der Sequenzen in Untergruppen, die die eindimensionalen Zusammensetzungsmetrikbereiche aufweisen, die mit Bereichen von menschlichen Genomdatenbankpartitionen identisch sind;
Zuordnen von Sequenzen in jeder der Sequenzuntergruppen zu geeigneten Untergruppen der partitionierten menschlichen Genomdatenbank;
Vergleichen der eindimensionalen Zusammensetzungsmetrikwerte mit ähnlichen Sequenzen in der vorpartitionierten menschlichen Genomdatenbank, um die Sequenzen aus einem metagenomischen Datensatz zu identifizieren, der sich mit den Fragmenten in der vorpartitionierten menschlichen Genomdatenbank mit einem vordefinierten Identitätswert ausrichtet;
Kategorisieren der ausgerichteten Fragmente als menschliche Genomverunreinigungen; und Entfernen identifizierter Verunreinigungssequenzfragmente aus der Mehrzahl von Sequenzfragmenten.

11. Nichtflüchtiges computerlesbares Medium, auf dem ein Computerprogramm zum Darstellen von Zusammensetzungseigenschaften von jedem der biologischen Sequenzfragmente unter Verwendung einer eindimensionalen Zusammensetzungsmetrik verkörpert ist, **gekennzeichnet durch** Erzeugen eines Satzes von räumlich gut getrennten Referenzvektoren in einem Merkmalsvektorraum, der zu den Zusammensetzungseigenschaften des biologischen Sequenzfragments gehört, zum Erzeugen einer eindimensionalen Zusammensetzungsmetrik; wobei zum Darstellen von Zusammensetzungseigenschaften von jedem biologischen Sequenzfragment unter Verwendung der eindimensionalen Zusammensetzungsmetrik das Computerprogramm prozessorimplementierte Schritte implementieren soll zum:

Sammeln einer Mehrzahl von biologischen Sequenzfragmenten unter Verwendung eines Sammelmoduls (202) für biologische Sequenzfragmente, wobei die Mehrzahl von biologischen Sequenzen aus einer Gruppe bestehend aus genomischen, metagenomischen oder Umweltproben gesammelt wird;

Sequenzieren der Mehrzahl von biologischen Sequenzfragmenten unter Verwendung eines Sequenziermoduls (204) für biologische Sequenzfragmente;

Erzeugen eines 256-dimensionalen Tetranukleotidfrequenzvektors (v), der jedem sequenzierten biologischen Sequenzfragment aus der Mehrzahl von sequenzierten biologischen Sequenzfragmenten entspricht;

Unterziehen der 256-dimensionalen Tetranukleotidfrequenzvektoren einer Hauptkomponentenanalyse (PCA);

Auswählen eines Paares von diskreten Vektoren, wobei jeder des Paares von diskreten Vektoren an Extrema einer ersten Hauptkomponente (PC 1) liegt und entlang PC1 maximal getrennt ist;

Wiederholen des Auswählens des Paares von diskreten Vektoren für jeden von PC2, PC3, ..., PCn, um in jeder Iteration zwei diskrete Vektoren zum Erzeugen eines ersten Satzes von Referenzvektoren unter Verwendung eines Referenzvektorerzeugungsmoduls (206) auszuwählen, wobei der erste Satz von Referenzvektoren Paare von diskreten Vektoren umfasst, die in einer Reihenfolge ihrer Auswahl angeordnet sind, wobei die Reihenfolge das Paar von diskreten Vektoren umfasst, das von den Extrema einer wichtigsten Hauptkomponente abgeleitet ist, die dem Paar von diskreten Vektoren vorausgeht, die von den Extrema von relativ weniger wichtigen Hauptkomponenten abgeleitet sind;

Berechnen der eindimensionalen Zusammensetzungsmetrik für jedes sequenzierte biologische Sequenzfragment aus der Mehrzahl von sequenzierten biologischen Sequenzfragmenten als eine kumulative Funktion eines Abstands des 256-dimensionalen Tetranukleotidfrequenzvektors (v), der einem individuellen biologischen Sequenzfragment aus den ersten drei oder mehr Referenzvektoren entspricht, die aus dem ersten Satz von Referenzvektoren ausgewählt sind, unter Verwendung eines Berechnungsmoduls (208) für eine eindimensionale Zusammensetzungsmetrik;

Trennen jedes sequenzierten biologischen Sequenzfragments aus der Mehrzahl von sequenzierten biologischen Sequenzfragmenten in eine Mehrzahl von Gruppen basierend auf einem jeweiligen Wert der eindimensionalen Zusammensetzungsmetrik unter Verwendung eines Trennmoduls (210) für sequenzierte biologische Sequenzfragmente; und

Verwenden der getrennten Mehrzahl von Gruppen der sequenzierten biologischen Fragmente zum Identifizieren einer oder mehrerer metagenomischer Sequenzen, die von menschlichen Verunreinigungen betroffen sind, wobei das Identifizieren der einen oder mehreren metagenomischen Sequenzen, die von menschlichen Verunreinigungen betroffen sind, umfasst:

Sortieren und Partitionieren der Sequenzen in Untergruppen, die die eindimensionalen Zusammensetzungsmetrikbereiche aufweisen, die mit Bereichen von menschlichen Genomdatenbankpartitionen identisch sind;

Zuordnen von Sequenzen in jeder der Sequenzuntergruppen zu geeigneten Untergruppen der partitionierten menschlichen Genomdatenbank;

Vergleichen der eindimensionalen Zusammensetzungsmetrikwerte mit ähnlichen Sequenzen in der vorpartitionierten menschlichen Genomdatenbank, um die Sequenzen aus einem metagenomischen Datensatz zu identifizieren, der sich mit den Fragmenten in der vorpartitionierten menschlichen Genomdatenbank mit einem vordefinierten Identitätswert ausrichtet;

Kategorisieren der ausgerichteten Fragmente als menschliche Genomverunreinigungen; und Entfernen identifizierter Verunreinigungssequenzfragmente aus der Mehrzahl von Sequenzfragmenten.

## Revendications

1. Procédé de représentation des propriétés de composition de chacun des fragments de séquence biologique à l'aide d'une métrique de composition unidimensionnelle, le procédé étant **caractérisé par** la génération d'un ensemble de vecteurs de référence correctement séparés spatialement dans un espace vectoriel caractéristique concernant lesdites propriétés de composition des fragments de séquence biologique, pour générer une métrique de composition unidimensionnelle ; dans lequel, pour représenter les propriétés de composition de chacun des fragments de séquence biologique à l'aide de la métrique de composition unidimensionnelle, le procédé comprend les étapes mises en oeuvre par processeur de :

collecte d'une pluralité de fragments de séquence biologique à l'aide d'un module de collecte de fragments de séquence biologique (202), la pluralité de séquences biologiques étant collectée parmi un groupe constitué d'échantillons génomiques, métagénomiques ou environnementaux ;

séquençage de la pluralité de fragments de séquence biologique à l'aide d'un module de séquençage de fragments de séquence biologique (204) ;

génération d'un vecteur de fréquence de tétranucléotide à 256 dimensions (v) correspondant à chaque fragment de séquence biologique séquencé parmi la pluralité de fragments de séquence biologique séquencés ;

soumission des vecteurs de fréquence de tétranucléotide à 256 dimensions à une analyse en composantes principales (ACP) ;

sélection d'une paire de vecteurs discrets, chacun de la paire de vecteurs discrets étant situé aux extrémités d'une première composante principale (PC1) et étant séparé de façon maximale le long de PC1 ;

répétition de la sélection de la paire de vecteurs discrets pour chacun de PC2, PC3,..., PCn, pour sélectionner deux vecteurs discrets dans chaque itération pour générer un premier ensemble de vecteurs de référence à l'aide d'un module de génération de vecteurs de référence (206), le premier ensemble de vecteurs de référence étant constitué des paires de vecteurs discrets agencées dans l'ordre de leur sélection, l'ordre comprenant la paire de vecteurs discrets dérivés des extrêmes des composantes principales les plus significatives précédant la paire de vecteurs discrets dérivés des extrêmes de composantes principales relativement moins significatives ;

calcul de la métrique de composition unidimensionnelle pour chaque fragment de séquence biologique séquencé parmi la pluralité de fragments de séquence biologique séquencés sous la forme d'une fonction cumulative d'une distance du vecteur de fréquence de tétranucléotide à 256 dimensions (v) correspondant à un fragment de séquence biologique individuel parmi les au moins trois premiers vecteurs de référence sélectionnés parmi le premier ensemble de vecteurs de référence à l'aide d'un module de calcul de métrique de composition unidimensionnelle (208) ;

ségrégation de chaque fragment de séquence biologique séquencé parmi la pluralité de fragments de séquence biologique séquencés en une pluralité de groupes sur la base de la valeur respective de la métrique de composition unidimensionnelle à l'aide d'un module de ségrégation de fragments de séquence biologique séquencés (210) ; et

utilisation de la pluralité de groupes ségrégés de fragments biologiques séquencés pour identifier une ou plusieurs séquences métagénomiques dépourvues de contaminants humains, l'identification des une ou plusieurs séquences métagénomiques dépourvues de contaminants humains comprenant :

tri et partitionnement des séquences en sous-groupes ayant des plages de métrique de composition unidimensionnelle identiques aux plages de partitions de bases de données de génome humain ;

mappage des séquences dans chacun des sous-groupes de séquences avec des sous-ensembles appropriés de la base de données du génome humain partitionnée ;

comparaison des valeurs de métrique de composition unidimensionnelle à des séquences similaires dans la base de données du génome humain prépartitionnée pour identifier les séquences provenant d'un ensemble de données métagénomiques, qui s'alignent sur les fragments dans la base de données du génome humain prépartitionnée avec une valeur d'identité prédéfinie ;

catégorisation des fragments alignés en tant que contaminants de génome humain ; et retrait des fragments de séquences contaminantes identifiés de la pluralité de fragments de séquences.

2. Procédé selon la revendication 1, dans lequel la métrique de composition unidimensionnelle est le score cmp, dans lequel le score cmp est calculé comme une distance cumulée entre le vecteur de fréquence de tétranucléotide à 256 dimensions (v) correspondant au fragment de séquence biologique individuel et chaque des au moins trois vecteurs de référence (rv1, rv2, rv3,... , rvN) en utilisant l'équation suivante score-cmp = dist (v-rv1) + dist (v-rv2) + dist (v-rv3)+...+dist (v- rvN), et le score cmp étant utilisé pour identifier un sous-ensemble de fragments d'ADN d'origine humaine contaminant des ensembles de données métagénomiques dérivés d'un hôte humain.

3. Procédé selon la revendication 1, dans lequel la distance entre le vecteur de fréquence de tétranucléotide à 256 dimensions (v) correspondant à chaque fragment de séquence biologique séquencé parmi la pluralité de fragments de séquence biologique séquencés est calculée à l'aide d'une métrique de distance sélectionnée dans un groupe comprenant la distance de Manhattan, la distance euclidienne et une métrique appropriée adaptée pour mesurer la distance dans un espace multidimensionnel.

**4.** Procédé selon la revendication 1, comprenant en outre la génération d'un vecteur de fréquence à n dimensions pour une pluralité de fréquences de k-mères, la pluralité de fréquences de k-mères étant différentes de la fréquence de tétranucléotide.

**5.** Procédé selon la revendication 1, dans lequel les vecteurs de référence constituent des vecteurs à 256 dimensions générés de manière aléatoire qui sont discrets dans l'espace vectoriel caractéristique.

**6.** Procédé selon la revendication 1, comprenant en outre le calcul de la métrique de composition unidimensionnelle pour chaque fragment de séquence biologique séquencé parmi la pluralité de fragments de séquence biologique séquencés sous la forme d'une fonction cumulative de la distance du vecteur de fréquence de tétranucléotide (v) correspondant à un fragment de séquence biologique individuel parmi les au moins trois premiers vecteurs de référence, les au moins trois vecteurs de référence étant dérivés d'un deuxième ensemble de vecteurs de référence.

**7.** Procédé selon la revendication 6, dans lequel la dérivation du deuxième ensemble de vecteurs de référence comprenant les étapes de génération d'un vecteur de fréquence de tétranucléotide à 256 dimensions (v) correspondant à une pluralité de fragments de séquence biologique générés de manière aléatoire d'une longueur prédéterminée, par soumission du vecteur de fréquence de tétranucléotide à 256 dimensions à une analyse en composantes principales (ACP) ; sélection de deux vecteurs qui sont situés aux extrémités de la première composante principale (PC1) et sont donc séparés de façon maximale le long de PC1 ; répétition de la sélection de deux vecteurs discrets pour chacun de PC2, PC3,..., PCn, de manière à sélectionner deux vecteurs discrets dans chaque itération pour générer le deuxième ensemble de vecteurs de référence, le deuxième ensemble de vecteurs de référence comprenant des paires de vecteurs discrets agencées dans l'ordre de leur sélection, dans un ordre dans lequel les paires de vecteurs de référence dérivées des extrêmes des composantes principales les plus significatives précèdent les paires de vecteurs de référence dérivées des extrêmes de composantes principales relativement moins significatives.

**8.** Procédé selon la revendication 6, dans lequel la pluralité de fragments de séquence biologique générés de manière aléatoire sont dérivés de génomes complètement séquencés.

**9.** Procédé selon la revendication 1, dans lequel la génération du vecteur de fréquence de tétranucléotide à 256 dimensions (v) correspondant à chaque fragment de séquence biologique séquencé parmi la pluralité de fragments de séquence biologique séquencés ; la soumission des vecteurs de fréquence de tétranucléotide à 256 dimensions à une analyse en composantes principales (ACP) ; la sélection de deux vecteurs qui sont situés aux extrêmes de la première composante principale (PC1) et sont donc séparés de façon maximale le long de PC1 ; la répétition de la sélection de deux vecteurs discrets pour chacun de PC2, PC3,..., PCn, de façon à sélectionner deux vecteurs discrets dans chaque itération pour générer le premier ensemble de vecteurs de référence à l'aide du module de génération de vecteurs de référence (206), dans lequel le premier ensemble de vecteurs de référence est constitué des paires de vecteurs discrets agencées dans l'ordre de leur sélection, dans l'ordre dans lequel les paires de vecteurs de référence dérivées des extrêmes de composantes principales les plus significatives précèdent les paires de vecteurs de référence dérivées des extrêmes de composantes principales relativement moins significatives, est un processus unique.

**10.** Système (200) pour représenter les propriétés de composition de chacun des fragments de séquence biologique à l'aide d'une métrique de composition unidimensionnelle, le procédé étant **caractérisé par** la génération d'un ensemble de vecteurs de référence correctement séparés spatialement dans un espace vectoriel caractéristique concernant lesdites propriétés de composition des fragments de séquence biologique, pour générer ladite métrique unidimensionnelle ; dans lequel, pour représenter les propriétés de composition de chacun des fragments de séquence biologique à l'aide de la métrique de composition unidimensionnelle, le système (200) comprend :

    a. un processeur ;
    b. un bus de données couplé audit processeur ;
    c. un support utilisable par ordinateur comportant un code informatique, ledit support utilisable par ordinateur étant couplé audit bus de données, ledit code de programme informatique comprenant des instructions exécutables par ledit processeur et configurées pour exécuter :

        un module de collecte de fragments de séquence biologique (202) adapté pour collecter une pluralité de fragments de séquence biologique, la pluralité de séquences biologiques étant collectée parmi un groupe constitué d'échantillons génomiques, métagénomiques ou environnementaux ;

un module de séquençage de fragments de séquence biologique (204) adapté pour séquencer la pluralité de fragments de séquence biologique ;

un module de génération de vecteurs de référence (206) adapté pour générer un vecteur de fréquence de tétranucléotide à 256 dimensions (v) correspondant à chaque fragment de séquence biologique séquencé parmi la pluralité de fragments de séquence biologique séquencés ; soumettre les vecteurs de fréquence de tétranucléotide à 256 dimensions à une analyse en composantes principales (ACP) ; sélectionner une paire de vecteurs discrets, la paire de vecteurs discrets étant située aux extrêmes d'une première composante principale (PC1) et étant séparée de façon maximale le long de PC1 ; répéter la sélection de la paire de vecteurs discrets pour chacun de PC2, PC3,..., PCn, pour sélectionner deux vecteurs discrets à chaque itération pour générer un premier ensemble de vecteurs de référence, le premier ensemble de vecteurs de référence étant constitué des paires de vecteurs discrets agencées dans l'ordre de leur sélection, l'ordre comprenant la paire de vecteurs discrets dérivés des extrêmes d'une composante principale la plus significative précédant la paire de vecteurs discrets dérivés des extrêmes de composantes principales relativement moins significatives ;

un module de calcul de métrique de composition unidimensionnelle (208) adapté pour calculer la métrique de composition unidimensionnelle pour chaque fragment de séquence biologique séquencé parmi la pluralité de fragments de séquence biologique séquencés sous la forme d'une fonction cumulative d'une distance du vecteur de fréquence de tétranucléotide à 256 dimensions (v) correspondant à un fragment de séquence biologique individuel parmi les au moins trois premiers vecteurs de référence sélectionnés parmi le premier ensemble de vecteurs de référence ; et un module de ségrégation de fragments de séquence biologique séquencés (210) adapté pour ségréger chaque fragment de séquence biologique séquencé parmi la pluralité de fragments de séquence biologique séquencés en une pluralité de groupes sur la base de la valeur respective de la métrique de composition unidimensionnelle, le système (200) étant adapté à l'aide de la pluralité de groupes ségrégés des fragments biologiques séquencés pour identifier une ou plusieurs séquences métagénomiques dépourvues de contaminants humains, l'identification des une ou plusieurs séquences métagénomiques dépourvues de contaminants humains comprenant :

tri et partitionnement des séquences en sous-groupes ayant des plages de métrique de composition unidimensionnelle identiques aux plages de partitions de bases de données de génome humain ;

mappage des séquences dans chacun des sous-groupes de séquences avec des sous-ensembles appropriés de la base de données du génome humain partitionnée ;

comparaison des valeurs de métrique de composition unidimensionnelle à des séquences similaires dans la base de données du génome humain prépartitionnée pour identifier les séquences provenant d'un ensemble de données métagénomiques, qui s'alignent sur les fragments dans la base de données du génome humain prépartitionnée avec une valeur d'identité prédéfinie ;

catégorisation des fragments alignés en tant que contaminants de génome humain ; et retrait des fragments de séquences contaminantes identifiés de la pluralité de fragments de séquences.

11. Support lisible par ordinateur non transitoire sur lequel est incorporé un programme informatique destiné à représenter les propriétés de composition de chacun des fragments de séquence biologique à l'aide d'une métrique de composition unidimensionnelle, **caractérisé par** la génération d'un ensemble de vecteurs de référence correctement séparés spatialement dans un espace vectoriel caractéristique concernant lesdites propriétés de composition du fragment de séquence biologique, pour générer une métrique de composition unidimensionnelle ; dans lequel, pour représenter les propriétés de composition de chaque fragment de séquence biologique à l'aide de la métrique de composition unidimensionnelle, le programme informatique doit mettre en oeuvre les étapes mises en oeuvre par processeur de :

collecte d'une pluralité de fragments de séquence biologique à l'aide d'un module de collecte de fragments de séquence biologique (202), la pluralité de séquences biologiques étant collectée parmi un groupe constitué d'échantillons génomiques, métagénomiques ou environnementaux ;

séquençage de la pluralité de fragments de séquence biologique à l'aide d'un module de séquençage de fragments de séquence biologique (204) ;

génération d'un vecteur de fréquence de tétranucléotide à 256 dimensions (v) correspondant à chaque fragment de séquence biologique séquencé parmi la pluralité de fragments de séquence biologique séquencés ;

soumission des vecteurs de fréquence de tétranucléotide à 256 dimensions à une analyse en composantes principales (ACP) ;

sélection d'une paire de vecteurs discrets, chacun de la paire de vecteurs discrets étant situé aux extrémités d'une première composante principale (PC1) et étant séparé de façon maximale le long de PC1 ;

répétition de la sélection de la paire de vecteurs discrets pour chacun de PC2, PC3,..., PCn, pour sélectionner deux vecteurs discrets dans chaque itération pour générer un premier ensemble de vecteurs de référence à l'aide d'un module de génération de vecteurs de référence (206), le premier ensemble de vecteurs de référence étant constitué des paires de vecteurs discrets agencées dans l'ordre de leur sélection, l'ordre comprenant la paire de vecteurs discrets dérivés des extrêmes d'une composante principale la plus significative précédant la paire de vecteurs discrets dérivés des extrêmes de composantes principales relativement moins significatives ;

calcul de la métrique de composition unidimensionnelle pour chaque fragment de séquence biologique séquencé parmi la pluralité de fragments de séquence biologique séquencés sous la forme d'une fonction cumulative d'une distance du vecteur de fréquence de tétranucléotide à 256 dimensions (v) correspondant à un fragment de séquence biologique individuel parmi les au moins trois premiers vecteurs de référence sélectionnés parmi le premier ensemble de vecteurs de référence à l'aide d'un module de calcul de métrique de composition unidimensionnelle (208) ;

ségrégation de chaque fragment de séquence biologique séquencé parmi la pluralité de fragments de séquence biologique séquencés en une pluralité de groupes sur la base de la valeur respective de la métrique de composition unidimensionnelle à l'aide d'un module de ségrégation de fragments de séquence biologique séquencés (210) ; et

utilisation de la pluralité de groupes ségrégés de fragments biologiques séquencés pour identifier une ou plusieurs séquences métagénomiques dépourvues de contaminants humains, l'identification des une ou plusieurs séquences métagénomiques dépourvues de contaminants humains comprenant :

tri et partitionnement des séquences en sous-groupes ayant des plages de métrique de composition unidimensionnelle identiques aux plages de partitions de bases de données de génome humain ;

mappage des séquences dans chacun des sous-groupes de séquences avec des sous-ensembles appropriés de la base de données du génome humain partitionnée ;

comparaison des valeurs de métrique de composition unidimensionnelle à des séquences similaires dans la base de données du génome humain prépartitionnée pour identifier les séquences provenant d'un ensemble de données métagénomiques, qui s'alignent sur les fragments dans la base de données du génome humain prépartitionnée avec une valeur d'identité prédéfinie ;

catégorisation des fragments alignés en tant que contaminants de génome humain ; et retrait des fragments de séquences contaminantes identifiés de la pluralité de fragments de séquences.

102

Collect a plurality of biological sequence fragments

104

Sequence the collected plurality of biological sequence fragments

106

Generating a first set of reference vectors

108

Compute a unidimensional compositional metric for each sequenced biological sequence fragment

110

Segregate each sequenced biological sequence fragment in to a plurality of groups based on respective unidimensional compositional metric

Figure 1

200

Biological sequence fragment collection module (202)

Biological sequence fragment sequencing module (204)

Reference vectors generation module (206)

Unidimensional compositional metric computation module (208)

Sequenced biological sequence fragment segregation module (210)

Figure 2

**One-time database creation steps**

Input Metagenomic Sequences

**302**

Generate 256 dimensional frequency vectors for each query sequence (qv) based on tetra-nucleotide usage

Human genome (database)

Splice into 500bp fragments (with 250bp overlaps)

Compute tetra-nucleotide frequencies of each database fragment to generate 256 dimensional frequency vectors (dv)

Select 3 frequency vectors which are spatially well-separated as reference vectors (rv)

Compute cmp-score for database fragments as:
$$cmp\text{-}score = \sum_{i=1}^{3} manhattan.\,dist(rv_i, dv)$$

Create 32 partitions of the database based on cmp-score values

**304**

Compute cmp-score for a query sequence as:
$$cmp\text{-}score = \sum_{i=1}^{3} manhattan.\,dist(rv_i, dv)$$

**306**

Query sequences partitioned into 32 subsets based on cmp-score values

**308**

Read mapping using BWA

Directed mapping against corresponding database partition(s)

Designate mapped reads as "Contaminating reads of human origin" and discard

Retain unmapped reads

Metagenomic sequences bereft of Human contaminants

**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 201621014353